# EUROPÄISCHE PATENTANMELDUNG

(11) **EP 4 507 133 A1**
(43) Veröffentlichungstag der Anmeldung: **12.02.2025**
(21) Anmeldenummer: 23190667.8
(22) Anmeldetag: 09.08.2023
(51) Int. Cl.: H01R 13/52, H01R 24/20, H01R 24/28, H01R 24/58, A61N 1/375, H01R 13/17, H01R 13/187, H01R 13/627

(54) **IMPLANTIERBARER ELEKTRISCHER STECKVERBINDER**

(71) Anmelder: Berlin Heart GmbH, 12247 Berlin (DE)
(72) Erfinder: Wange, Michael, 12247 Berlin (DE); Austilat, Konstantin, 12247 Berlin (DE)
(74) Vertreter: Pfenning, Meinig & Partner mbB

(57) **Zusammenfassung**

Die Erfindung bezieht sich auf einen implantierbaren elektrischen Steckverbinder (1) mit zwei zur elektrischen Kontaktgabe zusammenfügbaren Steckverbinderteilen (1a, 1b), von denen eines mit einer ersten Leitung (2a) und das andere mit einer zweiten Leitung (2b) verbunden ist, wobei jedes Steckverbinderteil wenigstens ein oder wenigstens zwei Kontaktpolteile (3a, 3b, 4a, 4b) jeweils in Form eines Kontaktstifts (3a, 3b) oder einer Kontaktbuchse (4a, 4b) aufweist, die jeweils mit Kontaktpolteilen des anderen Steckverbinderteils zur Kontaktgabe zusammensteckbar sind, wobei die Längsachsen (5a, 5b) der Kontaktpolteile (Steckrichtung der Kontaktpolteile in der diese zur Herstellung der Steckverbindung bewegt werden,) in einem Winkel (23) von wenigstens 30 Grad, insbesondere in einem Winkel von 90 Grad, zu einer Längsachse (6a, 6b) des ersten und/ oder des zweiten Steckverbinderteils verlaufen.

## Beschreibung

Die Erfindung liegt auf dem Gebiet der Medizintechnik, des Maschinenbaus und der Elektrotechnik und ist mit besonderem Vorteil bei Geräten und Vorrichtungen einsetzbar, die in den Körper eines Patienten implantierbar sind.

Es sind auf dem Gebiet der Medizintechnik verschiedene Geräte bekannt, die ganz oder teilweise in einen Patientenkörper implantierbar sind, wie beispielsweise Herzpumpen, Herzschrittmacher, Defibrillatoren und ähnliche Geräte. Viele solcher Geräte benötigen eine elektrische Energieversorgung, werden elektrisch gesteuert oder liefern Daten, die elektrisch weitergeleitet werden. In vielen Fällen müssen deshalb die Geräte über elektrische Leitungen angeschlossen oder mit anderen Aggregaten verbunden werden. Da für viele derartige Verbindungen eine relativ große Zahl von isolierten Einzelleitern notwendig ist, werden für die Herstellung von lösbaren Verbindungen relativ aufwändige Steckverbinder verwendet. Bekannt sind hierfür mehrpolige Verbinder mit parallelen Steckverbinderpolen, wie sie beispielsweise in dem US- Patent US10265450B2 beschrieben sind, oder auch sogenannte Mehrkanal Inline Steckverbinder, die im Wesentlichen mehrere koaxiale Kontakte aufweisen, die entlang einem Steckerstift oder einer Buchse hintereinander angeordnet sind. Derartige Steckverbinder sind in der Medizintechnik üblich und teilweise auch genormt.

Übersteigt die Anzahl der benötigten Einzelkontakte bestimmte Grenzen, so werden die Steckerstifte und Buchsen immer länger, so dass die derart aufgebauten Steckverbinder wegen ihres Platzbedarfs bei der Implantation unbrauchbar werden.

Der vorliegenden Erfindung liegt vor dem Hintergrund des Standes der Technik die Aufgabe zugrunde, einen Steckverbinder zu schaffen, der auch für eine Mehrzahl an Leitern eine einfache und sichere Steckverbindung bei möglichst geringem Platzbedarf bietet.

Die Aufgabe wird mit den Merkmalen der Erfindung gemäß einem der Patentansprüche 1 und 2 gelöst.

Demgemäß bezieht sich die Erfindung auf einen implantierbaren elektrischen Steckverbinder mit zwei zur elektrischen Kontaktgabe zusammenfügbaren Steckverbinderteilen, von denen eines mit einer ersten Leitung und das andere mit einer zweiten Leitung verbunden ist, wobei jedes Steckverbinderteil wenigstens ein oder wenigstens zwei Kontaktpolteile jeweils in Form eines Kontaktstifts oder einer Kontaktbuchse aufweist, die jeweils mit Kontaktpolteilen des anderen Steckverbinderteils zur Kontaktgabe zusammensteckbar sind,
wobei die Längsachsen der Kontaktpolteile in einem Winkel von wenigstens 30 Grad, insbesondere in einem Winkel von 90 Grad, zu einer Längsachse des ersten und/ oder des zweiten Steckverbinderteils verlaufen. Die Längsachsen der Kontaktpolteile definieren dabei die Steckrichtung der Kontaktpolteile in der diese zur Herstellung der Steckverbindung zusammengefügt werden. Die Längsachse eines Steckverbinderteils kann durch die Richtung der größten Längserstreckung eines Steckverbinderteils gegeben sein oder beispielsweise, wenn ein Steckverbinderteil ein zylindrisches Gehäuse aufweist, durch die Zylinderachse des Gehäuses. Der Winkel, den die Längsachsen der Kontaktpolteile und wenigstens eine der Längsachsen eines Steckverbinderteils einschließen und der wenigstens 30 Grad beträgt, ist der kleinere der beiden Winkel, die die genannten Achsen einschließen, so dass bei einer Bewegung der Steckverbinderteile während des Zusammensteckens in Steckrichtung entlang einer Längsachse der Kontaktpolteile die beiden Steckverbinderteile sich auch in Axialrichtung aneinander annähern, falls der Winkel kleiner als 90 Grad ist.

Dadurch, dass die Längsachsen der Kontaktpolteile mit einer Längsachse des ersten und/ oder des zweiten Steckverbinderteils einen Winkel von wenigstens 30 Grad, insbesondere 90 Grad einschließen, wird beim Zusammenstecken und Lösen der Steckverbindung deutlich weniger Platz benötigt als bei der bekannten Anordnung, bei der die Längsachen der Steckerpole parallel zur Längsachse eines oder mehrerer Steckverbinderteile verlaufen. In dem letztgenannten Fall addieren sich in der Position vor dem Zusammenstecken und nach dem Lösen der Steckverbinderteile die Längen der Kontaktpolteile und von Teilen der Steckverbindergehäuse sowie der Leitungsanschlüsse in Längsrichtung, so dass der Platzbedarf den in einem Patientenkörper zur Verfügung stehenden Platz deutlich übersteigt. Dies gilt insbesondere beim Einsatz derartiger Steckverbinder bei Kindern.

Weiter bezieht sich die Erfindung auf einen implantierbaren elektrischen Steckverbinder mit zwei zur elektrischen Kontaktgabe zusammenfügbaren Steckverbinderteilen von denen eines mit d einer ersten Leitung und das andere mit einer zweiten Leitung verbunden ist, wobei jedes Steckverbinderteil wenigstens ein oder wenigstens zwei Kontaktpolteile jeweils in Form eines Kontaktstifts oder einer Kontaktbuchse aufweist, die jeweils mit Kontaktpolteilen des anderen Steckverbinderteils zur Kontaktgabe zusammensteckbar sind, wobei die Längsachsen der Kontaktpolteile in einem Winkel von wenigstens 30 Grad, insbesondere in einem Winkel von 90 Grad, zu einer Längserstreckungsrichtung der ersten und/ oder der zweiten Leitung verlaufen.

Unter der Längserstreckungsrichtung der ersten und zweiten Leitung soll in diesem Zusammenhang die Längserstreckungsrichtung der jeweiligen Leitung unmittelbar im Anschlussbereich an das jeweilige Steckverbinderteil verstanden werden, oder auch die Austrittsrichtung der jeweiligen Leitung aus einem Steckverbinderteil. Auch in diesem Fall liegen die Längsachsen der Kontaktpolteile parallel zur Steckrichtung der Kontaktpolteile in der diese zur Herstellung der Steckverbindung zusammengefügt werden.

Der Winkel, den die Längsachsen der Kontaktpolteile und wenigstens eine der Längserstreckungsrichtungen der ersten und/ oder der zweiten Leitung einschließen und der wenigstens 30 Grad beträgt, ist der kleinere der beiden Winkel, die die genannten Achsen/Richtungen einschließen, so dass bei einer Bewegung der Steckverbinderteile während des Zusammensteckens in Steckrichtung entlang einer Längsachse der Kontaktpolteile die beiden Steckverbinderteile sich auch in Axialrichtung aneinander annähern, falls der Winkel kleiner als 90 Grad ist.

Der Platzbedarf kann in vielen Fällen bei einem derartigen Steckverbinder auch wenigstens teilweise durch die Leitungsanschlüsse und die angeschlossenen Leitungen bedingt sein.

In diesem Fall kann es, insbesondere dann, wenn wenigstens eine der Leitungen nicht parallel zu einer Längsachse des jeweiligen Steckverbinders, das heißt in der Verlängerung dieser Längsachse an diesen angeschlossen ist, vorteilhaft sein, wenn die Steckrichtung des Steckverbinders und die Richtung der Längsachsen der Kontaktpolteile nicht parallel zu der durch die Leitungsanschlüsse definierten Längsrichtung, also insbesondere zu einer Längserstreckungsrichtung der ersten und/ oder der zweiten Leitung verlaufen, sondern in einem Winkel von wenigstens 30 Grad zu dieser Richtung.

Bei einer besonderen Ausführungsform der Erfindung kann vorgesehen sein, dass an jedem der Steckverbinderteile wenigstens zwei, insbesondere wenigstens drei, weiter insbesondere wenigstens vier Kontaktpolteile nebeneinander vorgesehen sind, die zueinander parallele Längsachsen aufweisen.

Durch die Aufteilung der verschiedenen Kontaktverbindungen von separaten, voneinander elektrisch isolierten Leitungen auf verschiedene Kontaktpole entfällt auf jeden der Kontaktpole eine geringere Anzahl von Kontakten, so dass die Kontaktpole und damit die Kontaktpolteile, also die Kontaktstifte und die Buchsen, in Steckrichtung eine kürzere Baulänge aufweisen können, so dass der Steckverbinder bei der Herstellung und der Lösung der Kontakte weniger Platz beansprucht.

Bei einer weiteren Ausführungsform der Erfindung kann vorgesehen sein, dass wenigstens eines der Kontaktpolteile mehrere konzentrische, voneinander elektrisch isolierte Kontakte aufweist.

Derartige Kontaktpole mit konzentrischen Kontakten können besonders platzsparend aufgebaut werden.

Besonders platzsparend können solche Kontaktpole dadurch aufgebaut sein, dass jeweils wenigstens zwei der konzentrischen Kontakte in Längsrichtung des jeweiligen Kontaktpolteils gegeneinander versetzt sind. Es können in diesem Sinn auch mehr als zwei, insbesondere mehr als drei oder mehr als vier Kontakte in Längsrichtung an einem oder mehreren der Kontaktpole gegeneinander versetzt sein. Dies kann auch beispielsweise bei allen Kontaktpolen eines Steckverbinders der Fall sein.

Mit besonderem Vorteil kann auch vorgesehen sein, dass die Kontaktstifte oder die Kontaktbuchsen jeweils einen oder mehrere Ringfederkontakte aufweisen.

Solche Ringfederkontakte weisen jeweils die Form eines Torus auf, der durch eine Schraubenfeder gebildet ist. Die Ringfedern liegen im kontaktierten Zustand der Steckverbindung elastisch in Radialrichtung komprimiert zwischen einem Kontaktstift und einer Buchse und stellen auf diese Weise den elektrisch leitenden Kontakt zwischen diesen her.

Anstelle von Ringfederkontakten können auch andere, vorzugsweise federnde, Kontaktelemente zwischen einem Kontaktstift und einer Kontaktbuchse vorgesehen und in deren Längsrichtung verteilt sein.

Bei einem erfindungsgemäßen Steckverbinder kann zudem vorteilhaft vorgesehen sein, dass in Richtung der Längsachsen der Kontaktpolteile jeweils zwischen zwei Kontakten ein Dichtungsring angeordnet ist.

Diese Dichtungsringe können als Elastomerringe ausgebildet und beispielsweise jeweils zwischen zwei Ringfederkontakten angeordnet sein. Die Dichtungsringe können dadurch entlang einem Kontaktpol positioniert sein, dass sie in Nuten eines Kontaktstifts oder einer Kontaktbuchse liegen. Durch die Dichtungsringe werden Körperflüssigkeiten, die im implantierten Zustand trotz anderer Abdichtungsvorkehrungen in das Innere des Steckverbinders gelangen, daran gehindert, den Zwischenraum zwischen zwei Kontakten an einem Kontaktpol zu überbrücken und dadurch einen Kurzschluss zu erzeugen oder einen unerwünschten Kontakt herzustellen.

Bei einem elektrischen Steckverbinder mit zueinander konzentrischen Kontakten kann weiter vorgesehen sein, dass jeweils im zusammengesteckten Zustand ineinandergreifende Kontaktpolteile derart eingerichtet sind, dass ein oder mehrere Dichtungsringe, die einem der Kontaktpolteile zugeordnet sind, an Dichtflächen des jeweils anderen Kontaktpolteils dichtend anliegen, wobei die Dichtflächen insbesondere durch zylindrische Flächen von elektrisch isolierenden Hülsen gebildet sind.

Damit können an einem der Kontaktpolteile Dichtungsringe in bestimmten, definierten Abständen zueinander angeordnet sein, während an dem anderen, komplementären Kontaktpolteil jeweils in entsprechenden Abständen zwischen Kontakten Dichtflächen liegen, an denen die Dichtungsringe im zusammengesteckten Zustand der Steckverbindung fluiddicht anliegen.

Die Erfindung kann weiter dadurch ausgestaltet sein, dass die beiden Steckverbinderteile durch wenigstens einen beide Steckverbinderteile umfassenden elastischen Ring und/oder eine in Richtung der Längsachsen der Kontaktpolteile einrastende Rastverbindung zusammengehalten sind.

Durch derartige Halte- oder Befestigungselemente können die Steckverbinderteile platzsparend zusammengehalten werden, wobei auch zum Befestigen und Lösen der Befestigungselemente nur minimaler Raum notwendig ist.

Eine weitere Ausgestaltung der Erfindung kann vorsehen, dass jedes Paar von Kontaktpolteilen im zusammengesteckten Zustand von einem elastischen Kontaktpoldichtungsring umgeben ist, der zwischen dem ersten Steckverbinderteil und dem zweiten Steckverbinderteil angeordnet ist und an jedem der Steckverbinderteile dichtend anliegt, wobei insbesondere vorgesehen ist, dass die Kontaktpoldichtungsringe jeweils in eines der Steckverbinderteile integriert sind.

Ein solcher Kontaktpoldichtungsring liegt fluiddicht an beiden Steckverbinderteilen an und kann jeweils einen oder mehrere Kontaktpole umgeben. Damit wird im zusammengesteckten Zustand der Steckverbindung das Eindringen von Fluiden zu den beiden Kontaktpolteilen verhindert. Der Kontaktpoldichtungsring stellt somit eine Fluidbarriere dar, die vor den Dichtungsringen liegt, die zwischen den Kontakten angeordnet sind.

Weiter kann sich die Erfindung auch auf einen implantierbaren Steckverbinder der oben beschriebenen Art beziehen, wobei die Längsachsen der Kontaktpolteile in einem Winkel von wenigstens 30 Grad, insbesondere in einem Winkel von 90 Grad, zu einer Längsachse des ersten und/ oder des zweiten Steckverbinderteils oder auch parallel zur Längsachse des ersten und/oder des zweiten Steckverbinderteils verlaufen, wobei eines der Steckverbinderteile, das zumindest eine Kontaktbuchse aufweist, auf seiner Seite, die im zusammengesteckten Zustand des Steckverbinders dem anderen Steckverbinderteil zugewandt ist, eine wenigstens bereichsweise elastisch verformbare Dichtungshülse, insbesondere mit wenigstens einer Öffnung zur Aufnahme eines Kontaktstiftes, aufweist oder trägt.

Eine derartige Dichtungshülse kann die Kontaktpolteile zusätzlich zu anderen Abdichtungsmaßnahmen vor dem Eindringen von Flüssigkeiten schützen, die Fehlkontakte oder Kurzschlüsse erzeugen könnten. Durch die wenigstens teilweise elastische Ausgestaltung kann die Dichtungshülse wenigstens bereichsweise effektiv fluiddicht an anderen Bauteilen anliegen. Im zusammengesteckten Zustand des Steckverbinders liegt die Dichtungshülse in Axialrichtung und/oder Steckrichtung des Steckverbinders zwischen den beiden Steckverbinderteilen.

Der Steckverbinder kann dadurch vorteilhaft ausgestaltet werden, dass die Dichtungshülse aus einem, insbesondere biokompatiblen, Elastomer besteht.

In diesem Fall kann die Dichtungshülse als ganze homogen hergestellt werden und an ihrer gesamten Oberfläche Dichtflächen ausbilden, unter anderem auch zur Dichtung an einem in eine Öffnung eingeführten Kontaktstift.

Eine weitere Ausgestaltung des Steckverbinders kann vorsehen, dass eine oder mehrere Öffnungen der Dichtungshülse in jeweils einen Kanal zur Aufnahme eines Kontaktstifts münden, wobei die Kanäle jeweils koaxial zu Kontaktbuchsen des Steckverbinderteils verlaufen, das die Dichtungshülse aufweist und wobei wenigstens einer der Kanäle im Bereich seiner Öffnung eine Engstelle aufweist.

Im zusammengesteckten Zustand des Steckverbinders sind Kontaktstifte in die Öffnungen und durch die Kanäle in der dichtungshülse gesteckt und die Kanäle können an den Engstellen abgedichtet sein, dadurch dass der Durchmesser der Engstellen zumindest in einem in Axialrichtung komprimierten Zustand der Dichtungshülse geringer ist als der Durchmesser der Kontaktstifte und dass im Bereich der Engstellen das Material der Dichtungshülse durch die Kontaktstifte elastisch aufgeweitet ist und fluiddicht an den Kontaktstiften anliegt.

Der Steckverbinder kann weiter dadurch ausgestaltet werden, dass die Engstellen jeweils durch nach innen weisende Dichtlippen in den Kanälen gebildet sind.

Der Steckverbinder kann vorteilhaft auch derart gestaltet sein, dass die Dichtungshülse in dem Steckverbinder derart in Axialrichtung oder Steckrichtung des Steckverbinders komprimierbar ist, dass sie sich in den Richtungen senkrecht zur Axialrichtung oder Steckrichtung des Steckverbinders ausdehnt.

Beispielsweise können in an sich bekannter Weise die beiden Steckverbinderteile durch eine Überwurfmutter oder eine elastische Klammer in Axialrichtung zusammengepresst werden, wobei die Dichtungshülse in Axialrichtung komprimiert wird und sich in Querrichtung dazu ausdehnt. Damit können sich beispielsweise die Engstellen in den Kanälen verengen und Kontaktstifte eng umschließen. Die Dichtungshülse kann sich infolge der Ausdehnung auch au-ßen an ihrem Umfang an weitere Teile des Steckverbinders fluiddicht anlegen.

Im Folgenden wird die Erfindung anhand von Ausführungsbeispielen in Figuren einer Zeichnung gezeigt und nachfolgend erläutert.

Dabei zeigt
- Fig. 1: einen Steckverbinder gemäß dem Stand der Technik,
- Fig. 2: einen Steckverbinder gemäß der Erfindung in nicht zusammengestecktem Zustand der Steckverbinderteile,
- Fig. 3: den Steckverbinder aus Fig. 2 aus einer anderen Perspektive,
- Fig. 4: einen Steckverbinder gemäß der Erfindung im zusammengesteckten Zustand,
- Fig. 5: zwei Steckverbinderteile mit einem elastischen Ring sowie einer Rastverbindung vor dem Zusammenfügen,
- Fig. 6: die Elemente aus Figur 5 nach dem Zusammenfügen,
- Fig. 7: einen Längsschnitt durch zwei Steckverbinderteile gemäß der Erfindung,
- Fig. 8: zwei Steckverbinderteile, wobei die Kontaktstifte in voller Länge erkennbar sind,
- Fig. 9: die beiden Steckverbinderteile aus der Figur 8, wobei die Kontaktpoldichtringe an den Kontaktbuchsen erkennbar sind,
- Fign. 10, 11: schematisch die Winkel zwischen den Längsachsen der Kontaktpolteile und anderen Achsen des Steckverbinders, die gemäß der Erfindung vorteilhaft sind,
- Fig. 12: einen Steckverbinder mit einer Dichtungshülse zwischen den Steckverbinderteilen,
- Fig. 13: einen axialen Steckverbinder mit einer Dichtungshülse sowie
- Fig. 14: Details einer Dichtungshülse in einem Längsschnitt und in einer Frontalansicht.

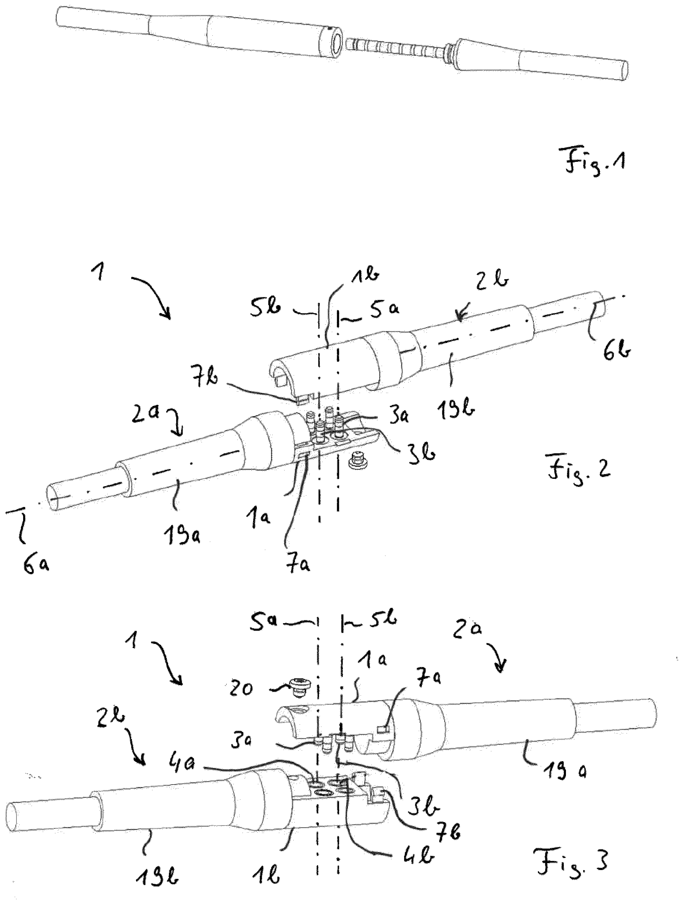

In der Figur 1 ist in einer perspektivischen Ansicht ein Steckverbinder gemäß dem Stand der Technik dargestellt. Ein Kontaktstift auf der rechten Seite der Darstellung weist mehrere konzentrische, in Längsrichtung des Kontaktstiftes gegeneinander versetzte elektrische Kontakte auf, die mit einzelnen Kontakten im Inneren der Kontaktbuchse auf der linken Seite der Darstellung zusammenwirken. Zum Lösen oder Stecken der Steckverbindung ist in axialer Richtung ein erheblicher Platzbedarf gegeben. Übersteigt die Anzahl der Kontakte eine bestimmte Grenze, so ist die Anwendbarkeit als implantierbarer Steckverbinder im Körper eines Patienten nicht mehr gegeben.

Die Figur 2 zeigt in einer perspektivischen Darstellung zwei Steckverbinderteile 1a, 1b im nicht zusammengesteckten Zustand. Der Steckverbinder 1 ist entlang einer Fläche geteilt, die parallel zu den Längsachsen 6a, 6b der Steckverbinderteile und der Leitungen 2a, 2b verläuft. Das in der Figur 2 unten dargestellte Steckverbinderteil 1a trägt Kontaktstifte 3a, 3b, deren Längsachsen 5a, 5b jeweils parallel zueinander verlaufen. Die Längsachsen 5a, 5b der Kontaktstifte verlaufen zudem senkrecht zu den Längsachsen 6a, 6b der Steckverbinderteile 1a, 1b. Das Zusammenstecken der beiden Steckverbinderteile 1a, 1b erfolgt nach einer anfänglichen Ausrichtung in Richtung parallel zu den Längsachsen 5a, 5b der Kontaktstifte 3a, 3b und der in der Figur 2 nicht dargestellten Kontaktbuchsen. Den Leitungen 2a, 2b wird auch jeweils ein an dem jeweiligen Steckverbinderteil 1a, 1b befestigter Knickschutz 19a, 19b als Teil der Leitungen zugerechnet.

Es ist zudem eine Befestigungsschraube 20 dargestellt sowie eine Rastverbindung, die zwei Rastnasen 7b und zugehörige Rastöffnungen 7a umfasst.

Grundsätzlich erfordert das Befestigen der Schraube 20 senkrecht zu den Längsachsen 6a, 6b der Steckverbinderteile einen seitlichen Zugang, während die Rastverbindung ohne weiteres Zutun beim Zusammenstecken der Steckverbinderteile einschnappt. Es kann deshalb sinnvoll sein, die Befestigungsschraube 20 in anderen Ausführungsformen zu vermeiden oder zu ersetzen.

Die Figur 3 zeigt in einer perspektivischen Darstellung die Steckverbinderteile 1a, 1b aus der Figur 2 aus einer anderen Blickrichtung. Es sind in der Figur 3 die Kontaktbuchsen 4a, 4b zuerkennen, in die die Kontaktstifte 3a, 3b entlang den Längsachsen 5a, 5b eingesteckt werden. Auch in der Figur 3 ist die Rastverbindung 7a, 7b und die Befestigungsschraube 20 zu erkennen.

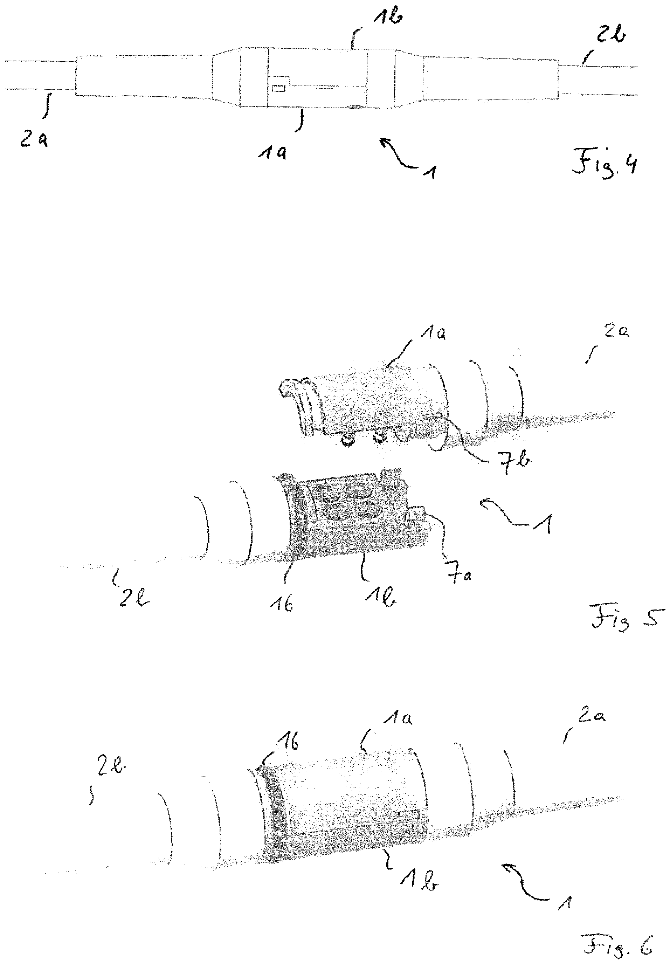

In der Figur 4 ist ein Steckverbinder mit zwei Steckverbinderteilen 1a, 1b im zusammengesteckten Zustand dargestellt. Bei dieser Ausführungsform verläuft die Längsachse des Steckverbinders parallel zu den Längsachsen der Leitungen 2a, 2b, die an diesen angeschlossen sind. Es ist jedoch auch denkbar, dass eine oder beide Längsachsen der Leitungen, die an den Steckverbinder angeschlossen sind, in einem spitzen Winkel zur Längsachse des Steckverbinders verlaufen. In diesem Fall können die Längsachsen der Kontaktpolteile beispielsweise senkrecht oder in einem Winkel zwischen 30° und 90° zu einer Längsachse von wenigstens einer der angeschlossenen Leitungen oder zur Längsachse des Steckverbinders ausgerichtet sein.

In der Figur 5 ist eine perspektivische Ansicht eines Steckverbinders 1a, 1a dargestellt, wobei die Steckverbinderteile in dieser Ausführungsform einerseits durch eine Rastverbindung 7a, 7b und andererseits durch einen elastischen Ring 16 zusammengehalten werden, der beide Steckverbinderteile umgibt.

Die Figur 6 zeigt die Steckverbinderteile 1a, 1b aus der Figur 5 im zusammengesteckten Zustand.

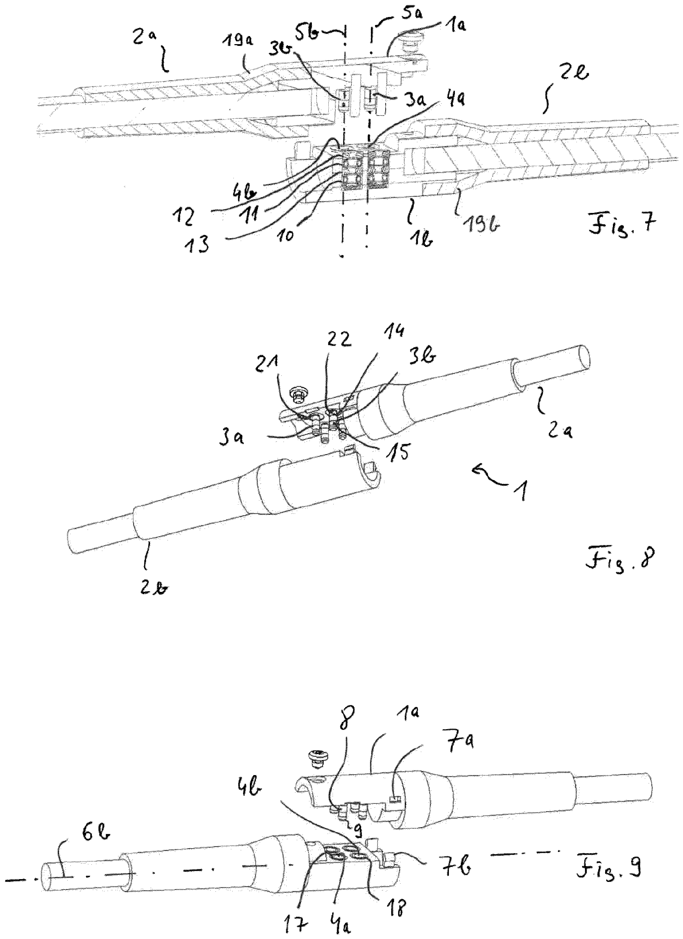

In der Figur 7 ist ein Längsschnitt durch zwei Steckverbinderteile dargestellt so dass eine Innenansicht der Kontaktbuchsen 4a, 4b erkennbar ist. Innerhalb der Kontaktbuchsen 4a, 4b sind in Richtung der Längsachsen 5a, 5b hintereinander mehrere Ringfederkontakte 10, 11 dargestellt, die jeweils mit Kontaktflächen 8, 9, der Kontaktstifte 3a, 3b zusammenwirken, die in der Figur 9 detaillierter dargestellt sind. Zwischen den Ringfederkontakten 10, 11 liegen Dichtungsringe 12, 13, die als Elastomerringe ausgeführt sein können. Die Elastomerringe 12, 13 dichten jeweils an Dichtflächen an den Kontaktstiften 3a, 3b die dort als isolierende Hülsen zwischen den Kontakten 8, 9 angeordnet und in der Figur 8 mit 14, 15 bezeichnet sind.

In der Figur 7 sind auf beiden Seiten der Steckverbinderteile 1a, 1b jeweils die Leitungen 2a, 2b gezeigt zu denen auch der jeweilige Knickschutz 19a, 19b zählt. Der Knickschutz 19a, 19b weist jeweils eine trichterförmige Erweiterung auf, die jeweils einen zylinderförmigen Stutzen des zugehörigen Steckverbinderteils umfasst.

In der Figur 8 sind jeweils an den Stellen, an denen die Kontaktstifte 3a, 3b aus dem Steckverbinderteil 1a austreten, ringförmige Nuten 21, 22 vorgesehen, die jeweils einen Kontaktstift konzentrisch umgeben. In diese Nuten 21, 22 greifen im zusammengesteckten Zustand des Steckverbinders Kontaktpoldichtringe 17, 18 ein und dichten somit den zylindrischen Raum um die Kontaktpolteile fluiddicht ab. Die Kontaktpoldichtringe 17, 18 sind in dem dargestellten Ausführungsbeispiel der Figur 9 in das Gehäuse des Steckverbinderteils 1b integriert und stehen über die Ränder der Kontaktbuchsen ein Stück weit in Richtung der Längsachsen der Kontaktpolteile vor. Damit wird ein zuverlässiges Anliegen der Kontaktpoldichtringe an beiden Steckverbinderteilen sichergestellt.

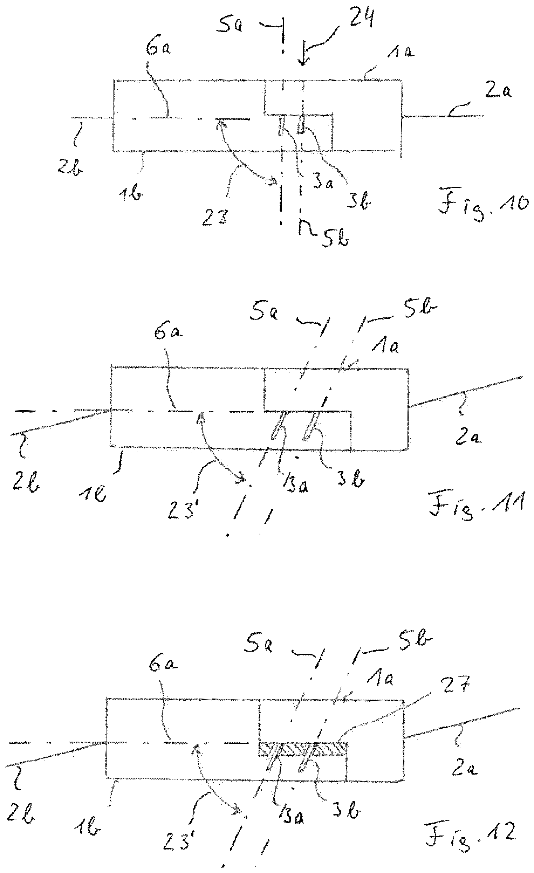

Die Figur 10 zeigt schematisch einen Steckverbinder im zusammengesteckten Zustand mit einer Längsachse 6a. Die angeschlossenen Leitungen 2a, 2b verlaufen ebenfalls in Richtung der Längsachse 6a. Die Längsachsen 5a, 5b der Kontaktstifte 3a, 3b verlaufen senkrecht zur Längsachse 6a, das heißt die beiden Längsachsen 6a und 5a, 5b schließen einen Winkel 23 von 90 Grad ein. Die Steckrichtung zum Zusammenstecken des Steckverbinders verläuft in Richtung der Längsachsen der Kontaktstifte oder der Kontaktpole und ist in der Figur 10 mit 24 bezeichnet.

In einer anderen denkbaren Ausführungsform gemäß Figur 11 stehen die zueinander parallelen Längsachsen 5a, 5b der Kontaktstifte 3a, 3b nicht senkrecht zur Längsachse eines Steckverbinderteils, sondern schließen mit der Längsachse 6a einen Winkel 23' ein, der von 30 Grad bis 90 Grad, insbesondere auch von 45 Grad bis 90 Grad betragen kann. Diese Winkelangabe bezieht sich jeweils auf den kleineren der beiden Winkel, der zwischen einer Längsachse der Kontaktpole und einer Längsachse eines Steckverbinderteils oder zwischen einer Längsachse der Kontaktpole und einer Längsrichtung einer der beiden an den Steckverbinder angeschlossenen Leitungen eingeschlossen wird, derart dass ein Einschieben der Kontaktstifte in die Kontaktbuchsen in den Fällen, in denen der Winkel 23 kleiner als 90 Grad ist, jeweils auch eine Annäherung der beiden Steckverbinderteile aneinander in deren Axialrichtung bedeutet.

Die Figur 12 zeigt einen Steckverbinder wie in der Figur 11 dargestellt, wobei jedoch das Steckverbinderteil 1b eine dichtungshülse 27 trägt. Diese ist im zusammengesteckten Zustand der Steckverbinderteile 1a, 1b zwischen diesen in der Steckrichtung, das heißt in Richtung der Längsachsen 5a, 5b der Kontaktpolteile, komprimiert und weist Öffnungen und Kanäle zur Aufnahme der Kontaktstifte 3a, 3b auf.

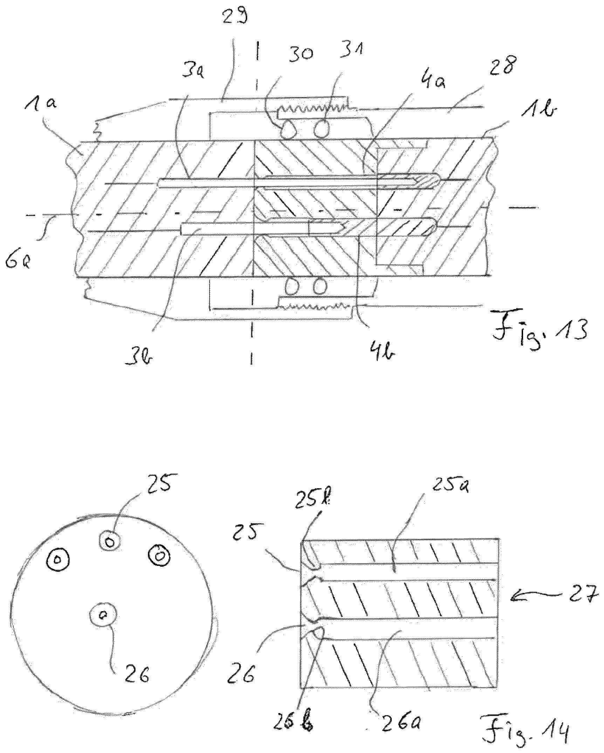

In der Figur 13 ist ein Teil eines Steckverbinders in einem Längsschnitt dargestellt, dessen Steckverbinderteile 1a, 1b in Richtung ihrer gemeinsamen Längsachse 6a zusammengesteckt werden. Die Steckverbinderteile können in diesem Fall beispielsweise bezüglich ihrer Außenkontur zylindersymmetrisch aufgebaut sein. Das Steckverbinderteil 1a weist zwei Kontaktstifte 3a, 3b auf, von denen beispielhaft der Kontaktstift 3a so lang gezeichnet ist, dass er in eine Kontaktbuchse 4a des Steckverbinderteils 1b hineinreicht. Die Kontaktbuchse 4a reicht dabei nicht in die Dichtungshülse 27 hinein, sondern endet axial vor dieser.

Der zweite Kontaktstift 3b erstreckt sich axial weniger weit über das Steckverbinderteil 1a hinaus als der Kontaktstift 3a und reicht in die Kontaktbuchse 4b hinein, die sich von dem Steckverbinderteil 1b aus axial bis in die Dichtungshülse 27 hinein erstreckt. Diese beiden Beispiele sollen für verschiedene mögliche Ausgestaltungen stehen, bei denen auch jeweils alle Kontaktstifte einerseits und alle Kontaktbuchsen andererseits gleich gestaltet sein können.

Mit dem Steckverbinderteil 1b ist eine Hülse 28 verbunden, die ein Außengewinde trägt. Auf diese ist eine Überwurfmutter 29 aufschraubbar, wodurch die beiden Steckverbinderteile 1a, 1b axial zusammenpressbar sind.

Im Zuge einer solchen Axialpressung wird auch die Dichtungshülse 27 axial komprimiert, so dass sie sich in radialer Richtung ausdehnt.

Hierdurch kann ein fluiddichtes Anliegen an den Dichtringen 30, 31 erzeugt oder unterstützt werden. Zudem können auch die Öffnungen und Kanäle im Inneren der Dichtungshülse radial komprimiert werden, die anhand der figur 14 weiter erläutert werden.

Die Figur 14 zeigt auf der rechten Seite einen Längsschnitt einer Dichtungshülse und auf der linken Seite eine Frontalansicht einer Dichtungshülse.

Im Längsschnitt sind die Kanäle 32, 33 erkennbar, die im Bereich der Öffnungen 34, 35 durch radial nach innen weisende Dichtlippen 32a, 33a, verengt sind. Die Verengungen sind so dimensioniert, dass Kontaktstifte die Dichtlippen beim Einstecken und Herausziehen gleitend berühren. Dadurch werden eventuell an den Kontaktstiften haftende Flüssigkeiten weitestgehend abgestreift, so dass die Kanäle flüssigkeitsfrei gehalten werden können.

## Patentansprüche

1. Implantierbarer elektrischer Steckverbinder (1) mit zwei zur elektrischen Kontaktgabe zusammenfügbaren Steckverbinderteilen (1a, 1b), von denen eines mit einer ersten Leitung (2a) und das andere mit einer zweiten Leitung (2b) verbunden ist, wobei jedes Steckverbinderteil wenigstens ein oder wenigstens zwei Kontaktpolteile (3a, 3b, 4a, 4b) jeweils in Form eines Kontaktstifts (3a, 3b) oder einer Kontaktbuchse (4a, 4b) aufweist, die jeweils mit Kontaktpolteilen des anderen Steckverbinderteils zur Kontaktgabe zusammensteckbar sind,
**dadurch gekennzeichnet, dass** die Längsachsen (5a, 5b) der Kontaktpolteile in einem Winkel (23) von wenigstens 30 Grad, insbesondere in einem Winkel von 90 Grad, zu einer Längsachse (6a, 6b) des ersten und/ oder des zweiten Steckverbinderteils verlaufen.

2. Implantierbarer elektrischer Steckverbinder (1) mit zwei zur elektrischen Kontaktgabe zusammenfügbaren Steckverbinderteilen (1a, 1b), von denen eines mit einer ersten Leitung (2a) und das andere mit einer zweiten Leitung (2b) verbunden ist, wobei jedes Steckverbinderteil wenigstens ein oder wenigstens zwei Kontaktpolteile (3a, 3b, 4a, 4b) jeweils in Form eines Kontaktstifts (3a, 3b) oder einer Kontaktbuchse (4a, 4b) aufweist, die jeweils mit Kontaktpolteilen des anderen Steckverbinderteils zur Kontaktgabe zusammensteckbar sind,
**dadurch gekennzeichnet, dass** die Längsachsen (5a, 5b) der Kontaktpolteile in einem Winkel (23) von wenigstens 30 Grad, insbesondere in einem Winkel von 90 Grad, zu einer Längserstreckungsrichtung der ersten und/ oder der zweiten Leitung (2a, 2b) verlaufen.

3. Elektrischer Steckverbinder nach Anspruch 1 oder 2, **dadurch gekennzeichnet, dass** an jedem der Steckverbinderteile (1a, 1b) wenigstens zwei, insbesondere wenigstens drei, weiter insbesondere wenigstens vier Kontaktpolteile (3a, 3b, 4a, 4b) nebeneinander vorgesehen sind, die zueinander parallele Längsachsen (5a, 5b) aufweisen.

4. Elektrischer Steckverbinder nach Anspruch 1, 2 oder 3, **dadurch gekennzeichnet, dass** wenigstens eines der Kontaktpolteile (3a, 3b, 4a, 4b) mehrere konzentrische, voneinander elektrisch isolierte Kontakte (8, 9) aufweist.

5. Elektrischer Steckverbinder nach Anspruch 4, **dadurch gekennzeichnet, dass** jeweils wenigstens zwei der konzentrischen Kontakte (8, 9) in Längsrichtung des jeweiligen Kontaktpolteils (3a, 3b, 4a, 4b) gegeneinander versetzt sind.

6. Elektrischer Steckverbinder nach einem der Ansprüche 1 bis 5, **dadurch gekennzeichnet, dass** die Kontaktstifte (3a, 3b) oder die Kontaktbuchsen (4a, 4b) jeweils einen oder mehrere Ringfederkontakte (10, 11) aufweisen.

7. Elektrischer Steckverbinder nach Anspruch 5 oder 6, **dadurch gekennzeichnet, dass** in Richtung der Längsachsen (5a, 5b) der Kontaktpolteile (3a, 3b, 4a, 4b) jeweils zwischen zwei Kontakten ein Dichtungsring (12, 13) angeordnet ist.

8. Elektrischer Steckverbinder nach Anspruch 1 oder einem der folgenden, **dadurch gekennzeichnet, dass** jeweils im zusammengesteckten Zustand ineinandergreifende Kontaktpolteile (3a, 3b, 4a, 4b) derart eingerichtet sind, dass ein oder mehrere Dichtungsringe (12, 13), die einem der Kontaktpolteile zugeordnet sind, an Dichtflächen (14, 15) des jeweils anderen Kontaktpolteils dichtend anliegen, wobei die Dichtflächen insbesondere durch zylindrische Flächen von elektrisch isolierenden Hülsen gebildet sind.

9. Elektrischer Steckverbinder nach einem der Ansprüche 1 bis 8, **dadurch gekennzeichnet, dass** die beiden Steckverbinderteile (1a, 1b) durch wenigstens einen beide Steckverbinderteile umfassenden elastischen Ring (16) und/oder eine in Richtung der Längsachsen (5a, 5b) der Kontaktpolteile (3a, 3b, 4a, 4b) einrastende Rastverbindung (7a, 7b) zusammengehalten sind.

10. Elektrischer Steckverbinder nach einem der Ansprüche 1 bis 9, **dadurch gekennzeichnet, dass** jedes Paar von Kontaktpolteilen(3a, 3b, 4a, 4b) im zusammengesteckten Zustand von einem elastischen Kontaktpoldichtungsring (17, 18) umgeben ist, der zwischen dem ersten Steckverbinderteil (1a) und dem zweiten Steckverbinderteil (1b) angeordnet ist und an jedem der Steckverbinderteile dichtend anliegt, wobei insbesondere vorgesehen ist, dass die Kontaktpoldichtungsringe jeweils in eines der Steckverbinderteile integriert sind.

11. Implantierbarer Steckverbinder nach Anspruch 1 oder einem der folgenden, wobei die Längsachsen (5a, 5b) der Kontaktpolteile in einem Winkel (23) von wenigstens 30 Grad, insbesondere in einem Winkel von 90 Grad, zu einer Längsachse (6a, 6b) des ersten und/ oder des zweiten Steckverbinderteils (1a, 1b) oder auch parallel zur Längsachse des ersten und/oder des zweiten Steckverbinderteils verlaufen, **dadurch gekennzeichnet, dass** eines der Steckverbinderteile, das zumindest eine Kontaktbuchse (4a, 4b) aufweist, auf seiner Seite, die im zusammengesteckten Zustand des Steckverbinders dem anderen Steckverbinderteil zugewandt ist, eine wenigstens bereichsweise elastisch verformbare Dichtungshülse (27), insbesondere mit wenigstens einer Öffnung (25, 26) zur Aufnahme eines Kontaktstiftes (3a, 3b), aufweist oder trägt.

12. Steckverbinder nach Anspruch 11, **dadurch gekennzeichnet, dass** die Dichtungshülse (27) aus einem, insbesondere biokompatiblen, Elastomer besteht.

13. Steckverbinder nach Anspruch 11 oder 12, **dadurch gekennzeichnet, dass** eine oder mehrere Öffnungen (25, 26) der Dichtungshülse (27) in jeweils einen Kanal (25a, 26a) zur Aufnahme eines Kontaktstifts münden, wobei die Kanäle jeweils koaxial zu Kontaktbuchsen (4a, 4b) des Steckverbinderteils (1a, 1b) verlaufen, das die Dichtungshülse aufweist und wobei wenigstens einer der Kanäle im Bereich seiner Öffnung eine Engstelle aufweist.

14. Steckverbinder nach Anspruch 11, 12 oder 13, **dadurch gekennzeichnet dass** die Engstellen jeweils durch nach innen weisende Dichtlippen (25b, 26b) in den Kanälen (25a, 26a) gebildet sind.

15. Steckverbinder nach einem der Ansprüche 11 bis 14, **dadurch gekennzeichnet, dass** die Dichtungshülse (27) in dem Steckverbinder derart in Axialrichtung (6a, 6b) oder Steckrichtung (24) des Steckverbinders komprimierbar ist, dass sie sich in den Richtungen senkrecht zur Axialrichtung oder Steckrichtung des Steckverbinders ausdehnt.
